# EUROPEAN PATENT APPLICATION

(11) **EP 2 982 465 A1**
(43) Date of publication of application: **10.02.2016**
(21) Application number: 14778905.1
(22) Date of filing: 03.04.2014
(51) Int. Cl.: B22F 9/24, A01N 25/12, A01N 59/16, A01P 3/00, B22F 1/00, B22F 1/02

(54) **METHOD FOR CONTROLLING PARTICLE SIZE OF SILVER PARTICLES, SILVER PARTICLES, ANTIMICROBIAL AGENT CONTAINING SILVER PARTICLES, AND USE THEREOF**

(30) Priority: 03.04.2013 JP 2013077823
(71) Applicant: SofSera Corporation, Tokyo 160-0022 (JP); Kinki University, Higashi-Osaka-shi Osaka 577-8502 (JP)
(72) Inventor: FURUZONO, Tsutomu, Kinokawa-shi Wakayama 649-6493 (JP); KOGAI, Yasumichi, Tokyo 160-0022 (JP); KAWABE, Karl Kazushige, Tokyo 160-0022 (JP)
(74) Representative: Bittner, Thomas L.
(86) International application number: PCT/JP2014/059803
(87) International publication number: WO 2014/163126

(57) **Abstract**

Silver particles are prepared by aggregating silver ions in accordance with a method including steps of (a), in preparing a mixed solution by adding a thiol compound to a solution containing silver ions, adjusting an amount of the thiol compound added to the solution and (b) adding a reducing agent to the mixed solution. This allows a particle size and antibacterial activity of the silver particles to be easily adjusted.

## Description

### Technical Field

The present invention relates to a method of controlling a particle size of silver particles, silver particles, an antibacterial agent containing silver particles, and use of silver particles.

### Background Art

It is widely known that silver has antibacterial activity. Currently, research is being carried out on a mechanism by which silver exhibits the antibacterial activity.

There are several theories about the mechanism by which silver exhibits the antibacterial activity, and the mechanism has not completely been clarified.

For example, Non-patent Literature 1 discloses the silver ion theory and the active oxygen theory as theories explaining the mechanism by which silver exhibits the antibacterial activity.

The silver ion theory suggests as follows. That is, by a substitution reaction, thiol groups (-SH) present in proteins of bacteria are replaced with silver ions. This causes inactivation of the proteins of the bacteria. As a result, the bacteria are inactivated.

On the other hand, according to the active oxygen theory, it is suggested that catalysis of silver ions causes generation of active oxygen and the active oxygen inhibits growth ability of bacteria.

Meanwhile, Non-Patent Literature 2 discloses another theory such that silver ions cause condensation of DNA of bacteria, thereby inhibiting growth ability of the bacteria.

The research is thus being carried on the mechanism of the antibacterial activity of silver. In the meanwhile, development of antibacterial agents, methods of eliminating bacteria, and the like with use of the antibacterial activity of silver are also being advanced.

For example, Patent Literature 1 discloses a method of producing fine particles having antibacterial activity which fine particles have a particle size of not more than 5 µm and which contain silver in a large amount. According to the method, silver halide fine particles are produced by mixing, in the presence of colloidal particles (for example, silicon oxide), a solution containing silver ions with a solution containing halide ions (for example, chloride ions, bromide ions, and iodide ions).

Under the circumstances, it is reported in recent years that, even in a case where silver particles are produced with use of identical materials, the silver particles vary in electric property, optical property, chemical property, and biological property depending on a size of the silver particles and as a result, the silver particles exhibit various behaviors.

For example, it is reported in Non-Patent Literature 3 that, in a case where silver particles have a nanometer particle size, antibacterial activity of the silver particles is enhanced and toxicity of the silver particles is significantly lower than that of silver chloride in an identical amount.

### Citation List

### [Patent Literature]

[Patent Literature 1]
Japanese Patent Application Publication Tokukai No. 2007-161649 (Publication date: June 28, 2007)

### [Non-patent Literature]

[Non-patent Literature 1]
   http://www.antimicrobial.co.jp/solution/About_Ag_antimicrobial_agent001.html
[Non-patent Literature 2]
   Q. L. Feng, J. Wu, G. Q. Chen, F. Z. Cui, T. N. Kim, J. O. Kim, Journal of Biomedical Materials Research, 52 (2000) 4
[Non-patent Literature 3]
   G. A. Martinez-Castanon et al., Journal of Nanoparticle Research, 10 (2008) 1343

### Summary of Invention

### Technical Problem

However, according to the conventional techniques, there have been problems that it is difficult to (i) adjust a particle size of silver particles to a desired size and (ii) adjust antibacterial activity of the silver particles to a desired level.

The present invention has been made in view of the above conventional problems, and an object of the present invention is to provide (i) a method of controlling a particle size of silver particles, which method allows a particle size of silver particles to be easily adjusted to a desired size and allows antibacterial activity of the silver particles to be easily adjusted to a desired level, (ii) silver particles, (iii) an antibacterial agent containing silver particles, (iv) and use of silver particles.

### Solution to Problem

In order to attain the above object, a method of controlling a particle size of silver particles of the present invention is a method of controlling a particle size of silver particles prepared by aggregating silver ions, the method including the steps of: in preparing a mixed solution by adding a thiol compound to a solution containing silver ions, adjusting an amount of the thiol compound added to the solution, the thiol compound being represented by a chemical formula HS-R-A (where R is any organic group, and A is any reactive functional group); and adding a reducing agent to the mixed solution.

The method of the present invention is preferably arranged such that a molarity of the thiol compound and a molarity of the silver ions of the mixed solution are adjusted so that the molarity of the thiol compound / the molarity of the silver ions > 1/1000.

The method of the present invention is preferably arranged such that the organic group R is an alkane, an alkene, or an alkyne.

The method of the present invention is preferably arranged such that the reactive functional group A is a carboxyl group, a sulphonyl group, a phosphate group, an alkoxysilyl group, a carboxylic acid anhydride, an amino group, or a hydroxyl group.

In order to attain the above object, silver particles of the present invention are silver particles including a chemically-modifying group represented by a chemical formula S-R-A (where R is any organic group, and A is any reactive functional group), the chemically-modifying group binding to at least part of a surface of particles containing silver.

The silver particles of the present invention are preferably arranged such that the organic group R is an alkane, an alkene, or an alkyne.

The silver particles of the present invention are preferably arranged such that the reactive functional group A is a carboxyl group, a sulphonyl group, a phosphate group, an alkoxysilyl group, a carboxylic acid anhydride, an amino group, or a hydroxyl group.

In order to attain the above object, an antibacterial agent of the present invention is an antibacterial agent including silver particles of the present invention.

The antibacterial agent of the present invention is preferably arranged such that a target is a fungus, a Gram-positive bacterium or a Gram-negative bacterium.

In order to attain the above object, a complex of the present invention is a complex including: silver particles of the present invention; and a base material to which the silver particles adhere.

The complex of the present invention is preferably arranged such that the silver particles bind to the base material through a chemical bond via a reactive functional group A.

In order to attain the above object, a medical device of the present invention is a medical device including a complex of the present invention.

### Advantageous Effects of Invention

A size of silver particles affects a level of antibacterial activity of the silver particles. Furthermore, an area of an exposed part of silver on a surface of the silver particles affects the level of the antibacterial activity of the silver particles. According to the present invention, not only a particle size of the silver particles is adjusted to a desired size, but also the area of the exposed part of silver on the surface of the silver particles is adjusted by binding a chemically-modifying group to at least part of a surface of particles containing silver. Therefore, according to the present invention, it is possible to (i) easily adjust the particle size of the silver particles to a desired size and (ii) easily adjust the antibacterial activity of the silver particles to a desired level.

According to the present invention, it is possible to adjust the particle size and the antibacterial activity of the silver particles at low cost with use of a simple device, instead of a large-sized device.

According to the present invention, it is possible to adjust the antibacterial activity of the silver particles to a desired level, thereby realizing silver particles safer for a living body (for example, a human). For example, in a case where the silver particles have too high antibacterial activity, the silver particles may affect the living body in various ways. According to the present invention, since it is possible to realize silver particles having proper antibacterial activity, it is possible to suppress only growth of bacteria without affecting the living body, even in a case where the silver particles comes into contact with the living body or enter the living body.

Since antibacterial effects of the silver particles of the present invention are varied depending on a type of bacteria, it is possible to selectively eliminate only targeted bacteria. For example, it is possible to eliminate only Gram-negative bacteria without eliminating Gram-positive bacteria. Obviously, it is possible to simultaneously eliminate both the Gram-negative bacteria and the Gram-positive bacteria. Moreover, since the antibacterial effects of the silver particles of the present invention are varied depending on a type of bacteria, it is also possible to identify bacteria (for example, it is possible to determine whether the bacteria are Gram-negative bacteria or Gram-positive bacteria). For example, it is possible to identify only Gram-positive bacteria present in a sample, by selectively eliminating only Gram-negative bacteria present in the sample.

According to the present invention, it is possible to realize excellent dispersibility of silver particles in a solution.

### Brief Description of Drawings

- Fig. 1: is a view illustrating an example process of forming silver particles of the present invention.
- Fig. 2: is a view illustrating an example structure of the silver particles of the present invention.
- Fig. 3: is a view illustrating a structural formula of 10-carboxy-1-decanthiol in an example of the present invention.
- Fig. 4: is a view illustrating IR spectra of silver particles of the example of the present invention.

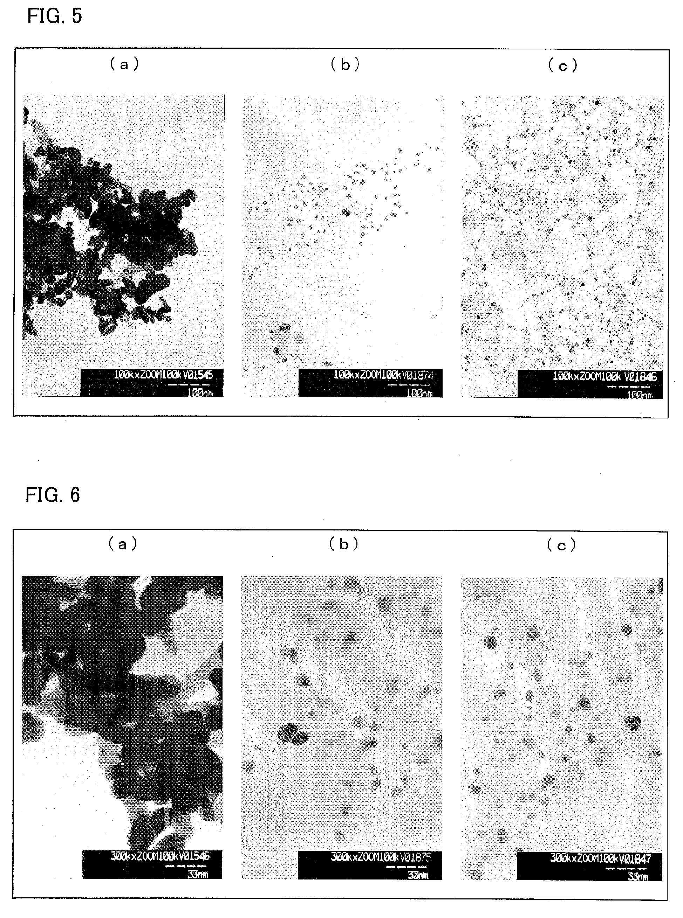

(a) through (c) of Fig. 5 each illustrate a picture, obtained by a transmission electron microscope (TEM), of the silver particles of the example of the present invention.

(a) through (c) of Fig. 6 each illustrate the picture, obtained by the transmission electron microscope (TEM), of the silver particles of the example of the present invention.

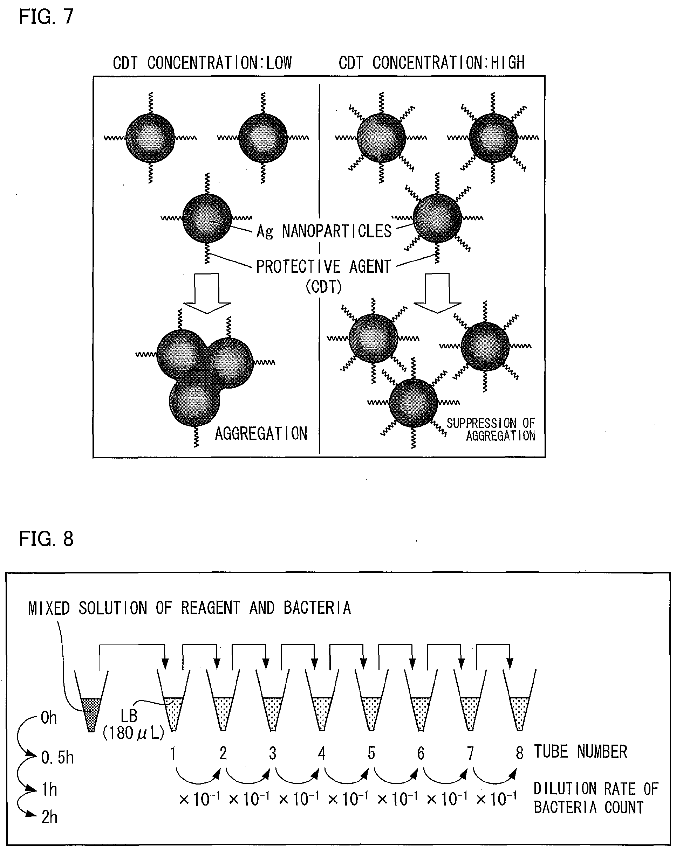

- Fig. 7: is a view explaining an assumed process of forming the silver particles of the example of the present invention.
- Fig. 8: is a view explaining a method of a test carried out in the example of the present invention.

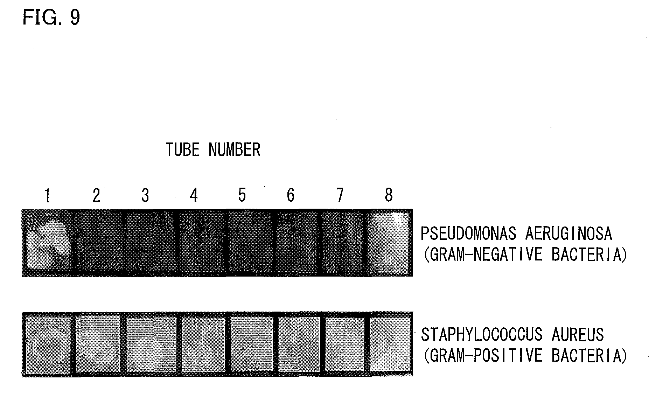

- Fig. 9: is a view illustrating results of the test carried out in the example of the present invention.

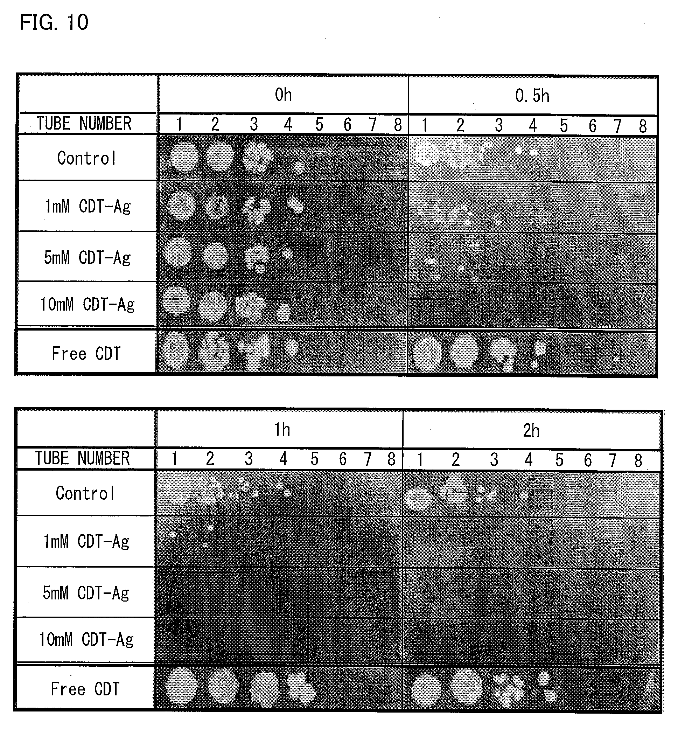

- Fig. 10: is a view illustrating results of a test carried out in the example of the present invention.

### Description of Embodiments

The following description will discuss an embodiment of the present invention. Please note, however, that the present invention is not limited to the embodiment.

### [1. Method of controlling particle size of silver particles]

A method of controlling a particle size of silver particles of the present embodiment is a method in which a particle size of silver particles, prepared by aggregating silver ions, is controlled.

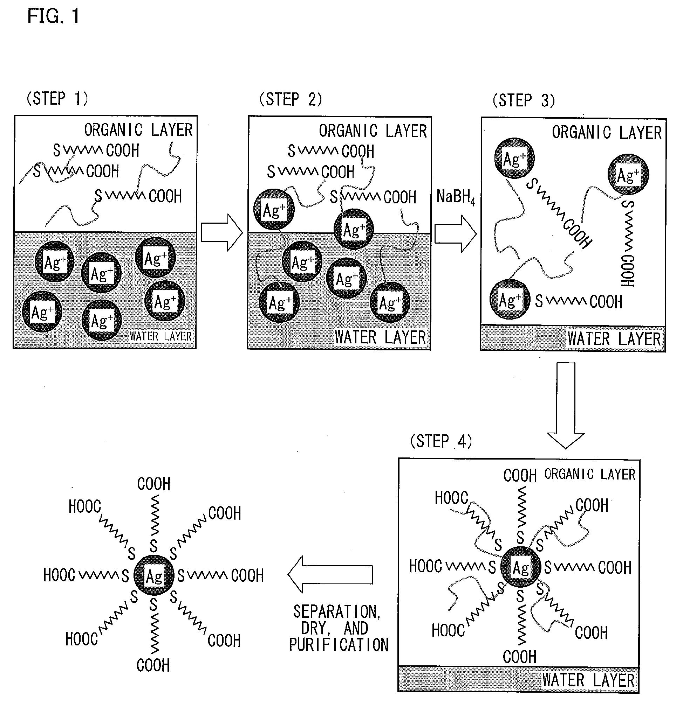

First, an outline of a process of forming the silver particles will be described with reference to Fig. 1. Note that the process illustrated in Fig. 1 is a mere example, and the present invention is not limited to such a process.

In the step 1, (i) an organic layer (for example, toluene) containing a thiol compound and (ii) a water layer containing silver ions are present in a state of being separated into two layers and being adjacent to each other. Note that, according to the method of the present embodiment, it is possible to adjust an amount of the thiol compound in the step 1. That is, according to the method of the present embodiment, the amount (concentration, in other words) of the thiol compound in the organic layer can be adjusted. Obviously, according to the method of the present embodiment, the amount of the thiol compound can be adjusted in a step other than the step 1.

In a step 2, the silver ions, contained in the water layer, move into the organic layer. In so doing, it is possible to promote such movement of the silver ions from the water layer to the organic layer by adding, to the organic layer, tetraoctylammonium=bromide, a phosphine ligand (triphenylphosphine), or a pyridinium ligand. Obviously, addition of such substances is not always necessary in the present invention.

In a step 3, a sulfur atom of the thiol compound binds to the silver ions.

In a step 4, the silver ions are reduced with use of a reducing agent (for example, NaBH₄) so as to produce silver particles.

In the present embodiment, the silver particles can be separated, dried and/or purified in a step subsequent to the step 4. However, such a step is not always necessary in the present invention.

The method of the present embodiment will be described below in more detail.

The method of the present embodiment includes a step of, in preparing a mixed solution by adding a thiol compound to a solution containing silver ions, adjusting an amount of the thiol compound added to the solution, the thiol compound being represented by a chemical formula HS-R-A (where R is any organic group, and A is any reactive functional group). This step causes the thiol compound to absorb to the silver ions.

According to the method of the present embodiment, in a case where the mixed solution is prepared by adding the thiol compound to the solution containing the silver ions, it is also possible to prepare the mixed solution by adding, to the solution (for example, water) containing the silver ions, a solution (for example, an organic solvent) containing the thiol compound.

The thiol compound can be directly added to the solution containing the silver ions. Alternatively, the thiol compound can be added to the solution containing the silver ions, after each of the silver ions contained in the solution is made a silver complex.

The silver complex is not limited to any particular one, and can be a desired one. For example, the silver complex can be a complex of a silver ion and tetraoctylammonium=bromide. Alternatively, the silver complex can be a complex of a silver ion and a phosphine ligand (for example, triphenylphosphine). Alternatively, the silver complex can be a complex of a silver ion and a pyridinium ligand. According to the above arrangement, it is possible to strengthen binding between silver and the thiol compound after nanoparticles, that is, the silver particles are formed.

The silver complex can be prepared, for example, by (i) mixing (a) a solution prepared by dissolving AgNO₃ in water with (b) a solution prepared by dissolving tetraoctylammonium=bromide, a phosphine ligand (triphenylphosphine), or a pyridinium ligand in an organic solvent (for example, toluene) and (ii) stirring a resultant mixed solution. Obviously, the present invention is not limited to such preparation. Note that it is possible to prepare the solution merely containing the silver ions each of which does not form the silver complex, by not mixing (a) the solution prepared by dissolving AgNO₃ in water with (b) the solution prepared by dissolving tetraoctylammonium=bromide, a phosphine ligand (triphenylphosphine), or a pyridinium ligand in an organic solvent

A solvent of the mixed solution of the silver complex and the thiol compound is not limited to any particular one. Examples of the solvent encompass water, an organic solvent (for example, toluene), and a mixture of water and an organic solvent (for example, toluene). However, the solvent is not limited to such examples.

The thiol compound added to the solution containing the silver ions can be represented by the chemical formula HS-R-A (where R is any organic group, and A is any reactive functional group).

The organic group R is not limited to any particular one. Examples of the organic group R encompass alkanes, alkenes, or alkynes. More specifically, the organic group R can be (CH₂)ₙ (where "n" is an integer of not less than 0 (zero)). Obviously, in the present invention, the organic group R is not limited such examples.

Alternatively, the organic group R can be any organic group having one or more double bond(s) between carbon atoms, any organic group having one or more triple bond(s) between carbon atoms, or any organic group having one or more double bond(s) between carbon atoms and one or more triple bond(s) between carbon atoms. In those cases, the organic group R can include an element other than carbon and hydrogen.

In a case where the organic group R is (CH₂)ₙ, the "n" is not limited to any particular integer. For example, the "n" can be an integer of not less than 0 (zero), not less than 5, not less than 10, or not less than 15. An upper limit of the "n" is not limited to any particular integer. For example, the upper limit can be 10, 20, or 30. More specifically, the "n" can be an integer between 0 (zero) and 10, an integer between 0 (zero) and 20, an integer between 0 (zero) and 30, an integer between 5 and 10, an integer between 5 and 20, an integer between 5 and 30, an integer of 10, an integer between 10 and 20, an integer between 10 and 30, an integer between 15 and 20, or an integer between 15 and 30. Obviously, the present invention is not limited to such integers.

According to the above arrangement, it is possible to effectively control a particle size of silver particles.

Out of the examples of the organic group R, a straight alkyl group is preferable. This is because, due to hydrophobic interaction between alkyl groups, it is possible to more effectively promote self-organization of the thiol compound and possible to stably maintain a structure of the silver particles after the silver particles are formed.

The reactive functional group A is not limited to any particular one. Examples of the reactive functional group A encompass carboxyl groups, sulphonyl groups, phosphate groups, alkoxysilyl groups, carboxylic acid anhydrides, amino groups, or hydroxyl groups.

According to the above arrangement, it is possible to bind the silver particles to various objects (for example, a medical device) through a chemical bond via the reactive functional group A. This allows the objects to have antibacterial activity.

Out of the foregoing examples of the reactive functional group A, a carboxyl group is preferable. This is because it is possible to easily make the chemical bond, such as an ester bond, while maintaining good dispersibility of the silver particles.

A more specific example of the thiol compound is one in which the organic group R is "(CH₂)₁₀" and the reactive functional group A is a carboxyl group, a sulphonyl group, a phosphate group, an alkoxysilyl group, a carboxylic acid anhydride, an amino group, or a hydroxyl group. Obviously, the present invention is not limited such an example.

Note that a thiol group (-HS) can be directly linked with the organic group R. Alternatively, the thiol group (-HS) can be indirectly linked with the organic group R via another element or another functional group. Similarly, the organic group R can be directly linked with the reactive functional group A. Alternatively, the organic group R can be indirectly linked with the reactive functional group A via another element or another functional group. In other words, it is sufficient that the thiol compound used in the method of the present embodiment is one that includes, in its structure, at least the thiol group, the organic group R, and the reactive functional group A.

According to the method of the present embodiment, the amount of the thiol compound added to the solution containing the silver ions is adjusted. This causes a concentration of the thiol compound in the mixed solution to be adjusted and causes an amount of the thiol compound which absorbs to the silver ions to be adjusted.

The amount of the thiol compound added to the solution containing the silver ions can be set as appropriate depending on the particle size and a level of antibacterial activity of the silver particles to be prepared. As the amount of the thiol compound added to the solution is smaller, the silver particles prepared have a larger particle size and lower antibacterial activity. In contrast, as the amount of the thiol compound added to the solution is larger, the silver particles prepared have a smaller particle size and higher antibacterial activity. Therefore, it is sufficient to set the amount of the thiol compound added to the solution containing the silver ions, depending on the particle size and the level of the antibacterial activity of the silver particles to be prepared.

For example, a ratio between a molarity of the thiol compound and a molarity of the silver ions (molarity of the thiol compound / molarity of silver ions) in the mixed solution containing the thiol compound and the silver ions can be not less than 1/1000, not less than 10/1000, not less than 100/1000, not less than 500/1000, not less than 1000/1000, not less than 5000/1000, or not less than 10000/1000. Note, however, that the ratio is not limited to such.

The method of the present embodiment includes a step of adding a reducing agent to the mixed solution of the silver ions and the thiol compound. This step causes the silver ions, to which the thiol compound absorbs, to be aggregated, so that the silver particles are produced. The reducing agent is not limited to any particular one. For example, the reducing agent can be sodium tetrahydroborate, lithiumtetrahydroaluminate, metallic sodium, or an ascorbic acid.

Out of examples of the reducing agent, sodium tetrahydroborate is preferable. This is because it is possible to easily control a reaction velocity and possible to more accurately prepare the silver particles having a desired particle size.

### [2. Silver particles]

Silver particles of the present embodiment are silver particles whose particle size is controlled by the method of controlling a particle size of silver particles of the present invention.

Specifically, the silver particles of the present embodiment are arranged such that a chemically-modifying group represented by a chemical formula S-R-A (where R is any organic group, and A is any reactive functional group) binds to at least part of a surface of particles containing silver.

More specifically, the silver particles of the present embodiment are arranged such that the chemically-modifying group represented by the chemical formula S-R-A (where R is any organic group, and A is any reactive functional group) binds, via sulfur, to at least part of the surface of the particles containing silver.

The organic group R is not limited to any particular one. Examples of the organic group R encompass alkanes, alkenes, or alkynes. More specifically, the organic group R can be (CH₂)ₙ (where "n" is an integer of not less than 0 (zero)). Obviously, in the present invention, the organic group R is not limited such examples.

Alternatively, the organic group R can be any organic group having one or more double bond(s) between carbon atoms, any organic group having one or more triple bond(s) between carbon atoms, or any organic group having one or more double bond(s) between carbon atoms and one or more triple bond(s) between carbon atoms. In those cases, the organic group R can include an element other than carbon and hydrogen.

In a case where the organic group R is (CH₂)ₙ, the "n" is not limited to any particular integer. For example, the "n" can be an integer of not less than 0 (zero), not less than 5, not less than 10, or not less than 15. An upper limit of the "n" is not limited to any particular integer. For example, the upper limit can be 10, 20, or 30. More specifically, the "n" can be an integer between 0 (zero) and 10, an integer between 0 (zero) and 20, an integer between 0 (zero) and 30, an integer between 5 and 10, an integer between 5 and 20, an integer between 5 and 30, an integer of 10, an integer between 10 and 20, an integer between 10 and 30, an integer between 15 and 20, or an integer between 15 and 30. Obviously, the present invention is not limited to such integers.

According to the above arrangement, it is possible to effectively control the particle size of the silver particles.

Out of the examples of the organic group R, a straight alkyl group is preferable. This is because, due to hydrophobic interaction between alkyl groups, it is possible to more effectively promote self-organization of the thiol compound and possible to stably maintain a structure of the silver particles after the silver particles are formed.

The reactive functional group A is not limited to any particular one. Examples of the reactive functional group A encompass carboxyl groups, sulphonyl groups, phosphate groups, alkoxysilyl groups, carboxylic acid anhydrides, amino groups, or hydroxyl groups.

According to the above arrangement, it is possible to bind the silver particles to various objects (for example, a medical device) through a chemical bond via the reactive functional group A. This allows the objects to have antibacterial activity.

Out of the foregoing examples of the reactive functional group A, a carboxyl group is preferable. This is because it is possible to easily make the chemical bond, such as an ester bond, while maintaining good dispersibility of the silver particles.

A more specific example of the chemically-modifying group is one in which the organic group R is "(CH₂)₁₀" and the reactive functional group A is a carboxyl group, a sulphonyl group, a phosphate group, an alkoxysilyl group, a carboxylic acid anhydride, an amino group, or a hydroxyl group. Obviously, the present invention is not limited such an example.

Note that, in the chemically-modifying group, sulfur can be directly linked with the organic group R. Alternatively, sulfur can be indirectly linked with the organic group R via another element or another functional group. Similarly, the organic group R can be directly linked with the reactive functional group A. Alternatively, the organic group R can be indirectly linked with the reactive functional group A via another element or another functional group. In other words, it is sufficient that the chemically-modifying group of the silver particles of the present embodiment is one that includes, in its structure, at least sulfur, the organic group R, and the reactive functional group A.

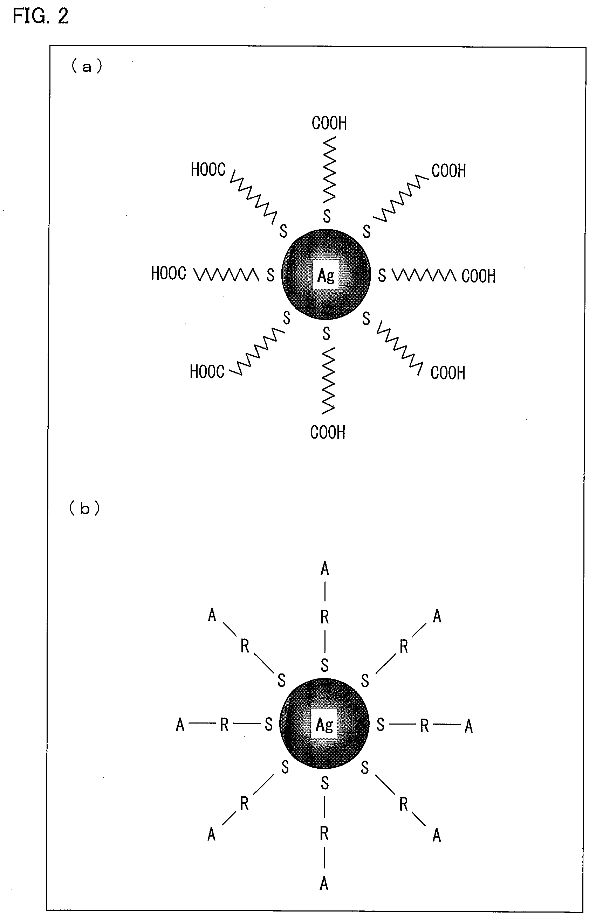

Fig. 2 illustrates an example silver particle of the present embodiment. According to the silver particle illustrated in Fig. 2, "S-(CH₂)₁₀-COOH" adheres, via "S," to a surface of aggregated silver. Note that the silver particles of the present embodiment are not limited to such. Alternatively, instead of "S-(CH₂)₁₀-COOH," various substances can adhere to the surface of aggregated silver.

The silver particles of the present embodiment are silver particles whose particle size is controlled by the method of the present invention. It is therefore possible to set the particle size to a desired size and possible to set antibacterial activity to a desired level.

For example, an average particle size of the silver particles of the present embodiment can be not more than 1000 nm, not more than 100 nm, not more than 50 nm, not more than 30 nm, not more than 26 nm, not more than 10 nm, not more than 7 nm, not more than 5 nm, or not more than 1 nm. A lower limit of the average particle size is not limited in particular. For example, the lower limit can be 0.1 nm, 0.5 nm, or 0.01 nm.

Note that it is possible to determine as appropriate the average particle size of the silver particles by a known method. For example, the average particle size can be determined by (i) measuring, with use of a ruler, diameters of the silver particles observed with use of a microscope and (ii) correct values thus measured in accordance with magnification of the microscope. Alternatively, the average particle size of the silver particles can be also determined with use of a laser diffraction/scattering particle size distribution measurement device (LMS-30, SEISHIN ENTERPRISE CO., LTD.). However, the present invention is not limited such methods.

The silver particles of the present embodiment are arranged such that the chemically-modifying group binds to at least part of the surface of the particles containing silver. A rate of the at least part of the surface of the particles containing silver, to which part the chemically-modifying group binds, is not limited to any rate. For example, the rate can be not less than 10%, not less than 20%, not less than 30%, not less than 40%, not less than 50%, not less than 60%, not less than 70%, not less than 80%, not less than 90%, or 100% of the surface.

In a case where the silver particles need to have as high antibacterial activity as possible, it is sufficient to reduce, as much as possible, the rate of the at least part of the surface of the particles containing silver, to which part the chemically-modifying group binds. In a case where the silver particles need to have as low antibacterial activity as possible, it is sufficient to increase, as much as possible, the rate of the at least part of the surface of the particles containing silver, to which part the chemically-modifying group binds.

From the other point of view, it is preferable that the antibacterial activity of the silver particles is not too low but not too high, in order to suppress toxicity of the silver particles to an object (for example, an animal cell) other than bacteria while suppressing growth of the bacteria. In this case, it is sufficient to bind the chemically-modifying group to 20% to 80%, 30% to 70%, 40% to 60%, or 50% of the surface of the particles containing silver. Obviously the present invention is not limited to such rates.

Note that it is possible to determine, as appropriate by a known method, the rate of the at least part of the surface of the particles containing silver, to which part the chemically-modifying group binds. For example, the rate can be determined by measuring a change in weight of the silver particles by a thermal analysis (TG-DTA). However, the present invention is not limited such a method.

Since silver has significantly high antibacterial activity, silver which is not covered by any chemically-modifying group or the like may affect a living body (for example, an animal such as a human) in various ways. Meanwhile, since the silver particles of the present embodiment are arranged such that at least part of the surface of the particles containing silver is covered by the chemically-modifying group, it is possible to adjust the antibacterial activity of the silver particles to a desired level. As a result, according to the silver particles of the present embodiment, it is possible to prevent silver from affecting the living body (for example, an animal such as a human) in various ways.

For example, according to the silver particles of the present embodiment, in a case where the silver particles are administered to a living tissue (for example, an animal tissue), it is possible to suppress growth of bacteria invading the living tissue or kill the bacteria, without damaging the living tissue.

Further, by coating, with the silver particles of the present embodiment, a surface of a medical device (for example, a catheter) disposed inside a living body, it is possible to prevent bacterial infection caused via the medical device, while securing safety of the living body.

### [3. Antibacterial agent]

An antibacterial agent of the present embodiment contains the silver particles of the present invention.

An amount of the silver particles contained in the antibacterial agent is not limited in particular. For example, the antibacterial agent can contain the silver particles in an amount of not less than 0.01% by weight, not less than 0.1% by weight, not less than 1% by weight, not less than 5% by weight, not less than 10% by weight, not less than 20% by weight, not less than 30% by weight, not less than 40% by weight, not less than 50% by weight, not less than 60% by weight, not less than 70% by weight, not less than 80% by weight, or not less than 90% by weight.

A target of the antibacterial agent of the present embodiment is not limited to any particular one. Examples of the target encompass fungi, Gram-positive bacteria (for example, *Staphylococcus* (such as *Staphylococcus aureus*), *Bacillus cereus*, *Streptococcus pyogenes*, and the like), and Gram-negative bacteria (for example, *Pseudomonas aeruginosa*, *Escherichia coli*, *Serratia*, *Salmonella*, and the like).

Since the antibacterial agent of the present embodiment has a high antibacterial effect on Gram-negative bacteria as shown in the example, it is possible to use the antibacterial agent of the present embodiment as an antibacterial agent targeted at the Gram-negative bacteria.

The antibacterial agent of the present embodiment can contain various substances (such as a solvent and a stabilizer) in addition to the silver particles. The substances are not limited in particular, and the antibacterial agent can contain various substances which are contained in known antibacterial agents.

The substances which can be contained in the antibacterial agent can be ones that specifically increase viability and/or proliferating ability of animal cells. In more detail, the substances can be ones that increase the viability and/or the proliferating ability of the animal cells and that do not increase viability and/or growth ability of microorganisms (such as fungi, Gram-positive bacteria, and Gram-negative bacteria).

The silver particles of the present invention can be arranged so as not to inhibit the viability and/or the proliferating ability of the animal cells. According to the above arrangement, in a case where the antibacterial agent is used for bodies of animals, it is possible to suppress only growth of microorganisms while more suppressing damage to cells of the animals.

Examples of the substances, which can be contained in the antibacterial agent, encompass a cell growth factor.

### [4. Complex and medical device]

A complex of the present embodiment is made up of the silver particles of the present invention and a base material to which the silver particles adhere.

Since the silver particles have been already described, description thereof will be omitted.

The base material is not limited to any particular one and can be, for example, one that is highly likely to be in contact with microorganisms. Not only the silver particles of the present invention are safe for a living body (for example, a human), but also the silver particles of the present invention have sufficient antibacterial activity. Therefore, by causing the silver particles to adhere to the base material which is highly likely to be in contact with microorganisms, it is possible to effectively suppress growth of the microorganisms or kill the microorganisms while securing safety of the living body.

Examples of the base material encompass medical devices (for example, a catheter, an endotracheal tube, a gastric fistula, an artificial blood vessel, an artificial joint, an artificial dental root, an artificial bone, an artificial pelvis, a bone-filling material, a wound dressing, a hernia repair mesh, a suture, an orthodontic wire, an orthopedic tape, a diagnostic drug carrier, and a mask). The base material can be the whole of a configuration of a medical device or part of the configuration of the medical device.

Among the configuration of the medical device, the base material can be one that is highly likely to be in contact with microorganisms. Examples of such configuration encompass an external wall of the medical device which external wall forms a boundary between the medical device and an outside (for example, air, a sample solution (such as blood and an infusion), and a living tissue) of the medical device. Examples of such configuration encompass an inner surface of a storage space provided inside the medical device for storage of a sample.

By selecting the medical device as the base material, it is possible to realize a medical device of the present invention.

### [Example]

### <1. Preparation of silver particles>

101.922 mg of silver nitrate was dissolved in 60 mL of ultrapure water to prepare a silver ion containing solution having a silver ion concentration of 10 mM.

656.172 mg of tetraoctylammonium=bromide was dissolved in 20 mL of toluene to prepare a tetraoctylammonium=bromide containing solution having a tetraoctylammonium=bromide concentration of 60 mM.

The silver ion containing solution and the tetraoctylammonium=bromide containing solution were mixed together in an eggplant-shaped flask. 120 mL of toluene was further added to a resultant mixed solution, and then the mixed solution thus obtained was stirred for 24 hours.

10-carboxy-1-decanthiol (CDT) was dissolved in 20 mL of toluene. Three types of protective agent containing solutions having CDT concentrations of 1 mM, 5mM, and 10 mM, respectively, were thus prepared.

After each of the protective agent containing solutions was independently added to the mixed solution, the mixed solution thus obtained was stirred for not less than 48 hours so as to cause 10-carboxy-1-decanthiol to adhere to silver ions.

226.98 mg of sodium tetrahydroborate (a reducing agent) was dissolved in 60 mL of ultrapure water to prepare a reducing agent containing solution having a sodium tetrahydroborate concentration of 100 mM.

The reducing agent containing solution was added to a mixture of the mixed solution and the each of the protective agent containing solutions. Thereafter, the mixture thus obtained was stirred for 1 (one) hour.

After being stirred, the mixture was left to stand until an organic layer and a water layer were phase-separated. By removing the water layer thus phase-separated, the organic layer was obtained. The organic layer was distilled under a reduced pressure with use of an evaporator so as to remove toluene. A solid which remained after removal of toluene was obtained.

The solid was suspended in methanol, vacuum-filtered, and then collected by centrifugation. Methanol was added to the solid thus collected, and a resultant mixture was subjected to ultrasonic irradiation so as to thoroughly disperse the solid.

Thereafter, by centrifugal separation (5000 rpm, 3 minutes), the solid thus dispersed in an aqueous solution was precipitated. After a supernatant solution was discarded, methanol was newly added to a precipitation. This operation was repeated three times.

The precipitation lastly obtained was dried under a reduced pressure, and then pulverized in a mortar. Silver particles were thus collected.

Note that, hereinafter, the silver particles prepared with use of the protective agent containing solution having the CDT concentration of 1 mM will be referred to as "1 mM CDT-Ag", the silver particles prepared with use of the protective agent containing solution having the CDT concentration of 5 mM will be referred to as "5 mM CDT-Ag", and the silver particles prepared with use of the protective agent containing solution having the CDT concentration of 10 mM will be referred to as "10 mM CDT-Ag".

### <2. Yield of silver particles, and organic component content rate of silver particles>

A yield of each of the foregoing three-types of silver particles was measured.

Further, an organic component content rate of each of the three-types of silver particles was measured.

The organic component content rate was measured as follows: (i) by a thermal analysis (TG-DTA), a change in weight of the silver particles between before and after heat treatment was measured; and (ii) the organic component content rate was calculated in accordance with the change in weight of the silver particles. More specifically, the weight of the silver particles at a time point when the silver particles were heated to approximately 500°C was subtracted from the weight of the silver particles at a time point when the silver particles were heated to approximately 200°C, and the organic component content rate was calculated in accordance with a change in weight thus obtained.

Results of measurement are shown in the following Table 1.

**[Table 1]**

| | 1 mM CDT-Ag | 5 mM CDT-Ag | 10 mM CDT-Ag |
|---|---|---|---|
| Yield (mg) | 35 | 60 | 102 |
| Organic Component Content rate (wt%) | 5.8 | 15.1 | 25.9 |

As is clear from Table 1, the three types of silver particles showed a tendency to become larger in yield, as the protective agent containing solutions used to prepare the respective three types of silver particles became higher in CDT concentration.

Furthermore, as is clear from Table 1, the three types of silver particles showed a tendency to become larger in organic component content rate, as the protective agent containing solutions used to prepare the respective three types of silver particles became higher in CDT concentration.

### <3. Evaluation of silver particles with Fourier transform infrared spectrophotometer>

Each of the three types of silver particles was evaluated with use of a Fourier transform infrared spectrophotometer (PerkinElmer, Inc.) in terms of a functional group present on a surface of particles containing silver.

Measurement was carried out by the KBr method. Specifically, 0.3 g of potassium bromide (KBr) powders were added to 0.003 g of each type of silver particles, and a resultant mixture was sufficiently ground in mortar. Uniformly mixed powders were measured in a diffuse reflection mode with a wavelength range from 4000 cm⁻¹ to 450 cm⁻¹ and with 4 times of integration.

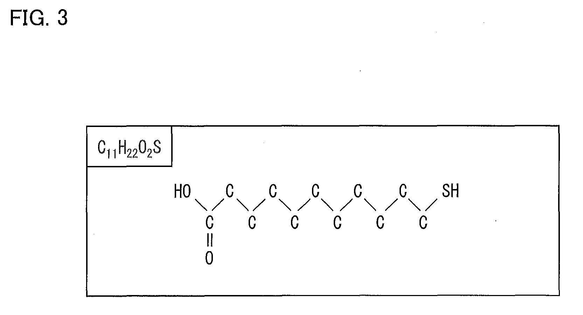

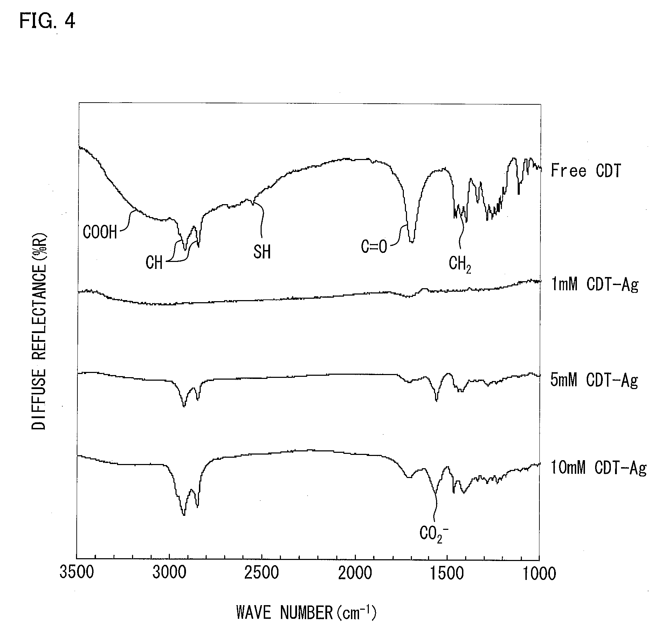

Fig. 3 illustrates a structural formula of CDT covering the particles containing silver. Fig. 4 illustrates results of the measurement.

"Free CDT" in Fig. 4 indicates an IR spectrum of CDT itself. As illustrated in Fig. 4, absorption was identified which was derived from each of "CH," "SH," and "C=0" included in a structure of CDT.

As illustrated in Fig. 4, it was found that the absorption derived from "CH" and the absorption derived from "C=0" increased in intensity in order of "1 mM CD-Ag," "5 mM CDT-Ag," and "10 mM CDT-Ag." That is, it was found that, as CDT used to prepare the silver particles became higher in concentration, the absorption derived from "CH" and the absorption derived from "C=0" increased in intensity.

Moreover, it was found from Fig. 4 that the absorption derived from "SH" disappeared according to the silver particles. This suggests that, in the silver particles, a sulfur atom binds to silver.

Furthermore, it was found from Fig, 4 that the absorption derived from "CO₂⁻" appeared according to the silver particles. This suggests that, in the silver particles, at least part of "COOH" is in a form of "COO⁻."

From the above test, it was found that the surface of the particles containing silver was covered by CDT. Further, it was found that, as CDT used to prepare the silver particles became higher in concentration, CDT contained in the silver particles per unit weight increased in amount.

### <4. Observation of form of silver particles with transmission electron microscope (TEM)>

With use of a transmission electron microscope (TEM), each form of the three types of silver particles was observed.

(a) of Fig. 5 is a TEM picture of "1 mM CDT-Ag." (b) of Fig. 6 is an enlargement of (a) of Fig. 5. (b) of Fig. 5 is a TEM picture of "5 mM CDT-Ag." (b) of Fig. 6 is an enlargement of (b) of Fig. 5. (c) of Fig. 5 is a TEM picture of "10 mM CDT-Ag." (c) of Fig. 6 is an enlargement of (c) of Fig. 5.

As is clear from (a) through (c) of Fig. 5 and (a) through (c) of Fig. 6, it was possible to observe the silver particles in each case. Further, it was found that, as CDT used to prepare the silver particles became higher in concentration, the silver particles increased in dispersibility.

Next, an average particle size of each of "1 mM CDT-Ag," "5 mM CDT-Ag," and "10 mM CDT-Ag" was calculated.

Specifically, 50 silver particles each having a substantially perfect circle were randomly extracted from each of the TEM pictures, and a diameter of each of the silver particles thus extracted was measured with use of a ruler. Then, an actual diameter of the each of the silver particles was calculated in accordance with (i) the diameter of the each of the silver particles measured with use of the ruler and (ii) magnification of the each of the TEM pictures. Thereafter, an average particle size of the silver particles was calculated in accordance with the actual diameter of the each of the silver particles. Table 2 shows the average particle size of each of the three types of silver particles.

**[Table 2]**

| | 1 mM CDT-Ag | 5 mM CDT-Ag | 10 mM CDT-Ag |
|---|---|---|---|
| Average Particles size (nm) | 26 | 10 | 7 |
| Standard Deviation (nm) | 7 | 3 | 2 |

As is clear from Table 2, as CDT used to prepare the silver particles became higher in concentration, the silver particles became smaller in average particle size.

This is assumed, as illustrated in Fig. 7, to be because, as the surface of the particles containing silver was covered by a larger amount of CDT, the silver particles were more effectively prevented from clustering in a large aggregate and, as a result, the silver particles became smaller in size.

### <5. Antibacterial test of silver particles-1>

Bacteria are unicellular organisms each having a size of approximately 0.2 µm to approximately 10 µm. Bacteria are larger in size than viruses, and each have a rigid cell wall.

Bacteria are roughly classified into two types by the Gram staining method. Bacteria which are stained violet by the Gram staining method are Gram-positive bacteria, whereas bacteria which are not stained violet but stained red by the Gram staining method are Gram-negative bacteria.

Such a difference in stainability results from a difference in cell wall structure. The Gram-positive bacteria each have a cell wall which includes a thick peptidoglycan layer and a few lipids, whereas the Gram-negative bacteria each have a cell wall which includes a thin peptidoglycan layer and many lipids. The difference in cell wall structure means that the Gram-positive bacteria are greatly different from the Gram-negative bacteria biologically.

In recent years, in-hospital infection caused via medical devices and the like has become a big problem. Examples of bacteria which cause the in-hospital infection encompass *Pseudomonas aeruginosa* (Gram-negative bacteria) and *Staphylococcus* (Gram-positive bacteria).

If it is possible to effectively prevent the in-hospital infection caused by such bacteria, this is of great significance in the medical field and the like. In view of this, the antibacterial activity of the silver particles of the present example was tested.

### <5-1. Test Method>

5 mg of each of "1 mM CDT-Ag," "5 mM CDT-Ag," and "10 mM CDT-Ag" was independently put in a tube. After methanol was added to the tube, a resultant mixture was subjected to ultrasonic irradiation so as to disperse the silver particles in methanol. Then, by centrifugal separation (5000 rpm, 3 minutes), the silver particles in an aqueous solution were precipitated. After the centrifugal separation, a supernatant in the tube was discarded, and precipitated silver particles remained in the tube was dried in a clean bench.

*Pseudomonas aeruginosa* (Gram-negative bacteria) having a spherical diameter of 1 mm or *Staphylococcus aureus* (Gram-positive bacteria) having a spherical diameter of 1 mm were suspended in a tube containing 1000 µL of an LB culture medium. A resultant mixture was treated by ultrasonication so that the bacteria were dispersed in the LB culture medium.

200 µL of the LB culture medium in which the bacteria were dispersed was added to the tube containing the precipitated silver particles, and then mixed with the precipitated silver particles to prepare a mixed solution containing the silver particles and the bacteria.

A time when the silver particles were mixed with the bacteria was assumed to be "0 (zero) h (hour)." Then, the following operations A) through K) were further carried out. Note that an outline of the operations A) through K) is shown in Fig. 8.
A) To each of new eight tubes, 180 µL of an LB culture medium was added. The tubes were numbered from "1" through "8" (thereinafter, referred to as a tube 1, a tube 2, a tube 3 ...).
B) 20 µL of the mixed solution containing the silver particles and the bacteria was added to the tube 1, and a resultant mixture was stirred.
C) The tube 1 was stored in an incubator whose inner temperature was set to 37°C, while being mixed upside down.
D) 20 µL of the LB culture medium was taken out from the tube 1, and added to the tube 2. As a result, a concentration of the bacteria in the tube 2 became one tenth of the concentration of the bacteria in the tube 1. The tube 2 was stored in the incubator whose inner temperature was set to 37°C, while being mixed upside down.
E) 20 µL of the LB culture medium was taken out from the tube 2, and added to the tube 3. As a result, the concentration of the bacteria in the tube 3 became one tenth of the concentration of the bacteria in the tube 2. The tube 3 was stored in the incubator whose inner temperature was set to 37°C, while being mixed upside down.
F) 20 µL of the LB culture medium was taken out from the tube 3, and added to the tube 4. As a result, the concentration of the bacteria in the tube 4 became one tenth of the concentration of the bacteria in the tube 3. The tube 4 was stored in the incubator whose inner temperature was set to 37°C, while being mixed upside down.
G) 20 µL of the LB culture medium was taken out from the tube 4, and added to the tube 5. As a result, the concentration of the bacteria in the tube 5 became one tenth of the concentration of the bacteria in the tube 4. The tube 5 was stored in the incubator whose inner temperature was set to 37°C, while being mixed upside down.
H) 20 µL of the LB culture medium was taken out from the tube 5, and added to the tube 6. As a result, the concentration of the bacteria in the tube 6 became one tenth of the concentration of the bacteria in the tube 5. The tube 6 was stored in the incubator whose inner temperature was set to 37°C, while being mixed upside down.
I) 20 µL of the LB culture medium was taken out from the tube 6, and added to the tube 7. As a result, the concentration of the bacteria in the tube 7 became one tenth of the concentration of the bacteria in the tube 6. The tube 7 was stored in the incubator whose inner temperature was set to 37°C, while being mixed upside down.
J) 20 µL of the LB culture medium was taken out from the tube 7, and added to the tube 8. As a result, the concentration of the bacteria in the tube 8 became one tenth of the concentration of the bacteria in the tube 7. The tube 8 was stored in the incubator whose inner temperature was set to 37°C, while being mixed upside down. Note that the concentration of the bacteria in the tube 8 can be assumed to be approximately 0 (zero).
K) 5 µL of the LB culture medium was taken out from each of the tubes 1 through 8, and independently spotted on an agar culture medium in a petri dish. The petri dish was covered, and then stored, so as to incubate the bacteria, in the incubator whose inner temperature was set to 37°C.

"0.5 h," "1 (one) h," and "2 h" after the silver particles were mixed with the bacteria, the LB culture medium was taken out from each of the tubes 1 through 8 stored in the incubator, and the operation K) was repeated

### <5-2. Test results>

Fig. 9 illustrates results of the test carried out with use of "1 mM CDT-Ag." More specifically, Fig. 9 illustrates results of, "2 h" after the silver particles were mixed with the bacteria, (i) taking out the LB culture medium from each of the tubes 1 through 8 stored in the incubator and (ii) carrying out the operation K), that is, results of incubating the bacteria at 37°C for 24 hours by storing the petri dish, prepared in the operation K), in the incubator.

As illustrated in Fig. 9, in a case of the *Pseudomonas aeruginosa*, growth of the bacteria was observed in the tube 1. However, the growth of the bacteria was not observed in each of the tubes 2 through 8. On the other hand, in a case of the *Staphylococcus aureus*, growth of the bacteria was observed in each of the tubes 1 through 4. However, the growth of the bacteria was not observed in each of the tubes 5 through 8.

From the above test results, it was found that the silver particles of the present invention have antibacterial activity for both Gram-negative bacteria and Gram-positive bacteria. Moreover, from the test results, it was found that the antibacterial activity of the silver particles of the present invention for the Gram-negative bacteria is 1000 times higher than that for the Gram-positive bacteria. That is, it was found that the silver particles of the present invention have higher antibacterial activity for the Gram-negative bacteria than for the Gram-positive bacteria.

### <6. Antibacterial test of silver particles-2>

A test similar to that in <5. Antibacterial test of silver particles-1> was carried out with use of *Escherichia coli* (Gram-negative bacteria), which is one of model organisms and by which bacteria are typified. Note that the test was carried out by a method similar to that in <5. Antibacterial test of silver particles-1>, except that a kind of the bacteria used in the test was changed. Therefore, the method of the test will not be described here, and only results of the test will be described.

Fig. 10 illustrates results of the test carried out with use of "1mM CDT-Ag," "5 mM CDT-Ag," and "10 mM CDT-Ag." More specifically, Fig. 10 illustrates results of, "0 (zero) h," "0.5 h," "1 (one) h," and "2 h" after the silver particles were mixed with the bacteria, (i) taking out an LB culture medium from each of tubes 1 through 8 stored in an incubator and (ii) carrying out an operation K), that is, a result of incubating the bacteria at 37°C for 24 hours by storing a petri dish, prepared in the operation K), in the incubator.

Further, the following Table 3 shows a bacteria count in the "tube 1" after "0.5 h," the bacteria count in the "tube 1" after "1 (one) h," and the bacteria count in the "tube 1" after "2 h," each illustrated in Fig. 10, assuming that the bacteria count in the "tube 1" after "0 (zero) h" illustrated in Fig. 10 is 1 (one).

**[Table 3]**

| | 0 h | 0.5 h | 1 h | 2 h |
|---|---|---|---|---|
| Control | 1 | 1 | 1 | 1 |
| 1 mM CDT-Ag | 1 | 10⁻¹ | 10⁻² | 0 |
| 5 mM CDT-Ag | 1 | 10⁻² | 0 | 0 |
| 10 mM CDT-Ag | 1 | 0 | 0 | 0 |
| Free CDT | 1 | 1 | 1 | 1 |

As is clear from Fig. 10 and Table 3, it was found that, as CDT used to prepare the silver particles became higher in concentration, the bacteria died out in a shorter time period. That is, it was found that the silver particles prepared with use of CDT in higher concentration had higher antibacterial activity.

Moreover, as is clear from Fig. 10 and Table 3, it was found that CDT itself did not have antibacterial activity.

### <7. Antibacterial test of silver particles-3>

"5mM CDT-Ag" prepared in <1. Preparation of silver particles> was fixed on a silicon sheet via an amide bond, and antibacterial activity of the silicon sheet for various bacteria was tested. A test method and a test result will be described below.

### <7-1. Preparation of silicon sheet on which silver particles are fixed>

### (A. graft polymerization)

First, an acrylic acid was graft-polymerized on the silicon sheet so as to introduce a carboxyl group to a surface of the silicon sheet. A procedure of this test will be described below.

A surface of a silicon sheet (SR-S 0.3×500×500BA (Shin-Etsu Polymer Co., Ltd.)) was treated more than once with corona discharge at 100 V for 15 seconds each so that radicals were generated on the surface of the silicon sheet.

The silicon sheet on which the radicals were generated was put in a test tube, and the test tube was heated with use of a gas burner so as to have a narrow portion.

After the test tube was cooled down, 10% of acrylic acid (monomer (Nacalai Tesque, Inc.)) was poured into the test tube.

After air in the test tube was replaced with nitrogen, the test tube was vacuumized with use of a vacuum pump.

After the test tube was vacuumized, the test tube was burned off at the narrow portion with use of the gas burner. The test tube was allowed to stand still in a water bath at 60°C for 30 minutes so that the acrylic acid was polymerized.

The test tube was cut open, and the silicon sheet was taken out into a beaker. The silicon sheet was then washed with running water for 40 minutes.

In a state of being soaked in ultrapure water, the silicon sheet was washed for 1 (one) day while being stirred. Thereafter, the silicon sheet was air-dried.

The silicon sheet having the surface to which a carboxyl group was introduced was thus obtained.

### (B. Introduction of amino group)

A polyacrylic acid was graft-polymerized on the surface of the silicon sheet as described above so as to introduce the carboxyl group to the surface of the silicon sheet. Next, an amino group of polyallylamine was reacted with the carboxyl group on the silicon sheet so as to form an amide bond and introduce the amino group to the surface of the silicon sheet. A method of introducing the amino group to the surface of the silicon sheet will be described below.

0.0135g of WSC (EDC/EDAC (Wako Pure Chemical Industries Ltd.)) was dissolved in 300 mL of ion exchanged water, and a resultant solution was stirred for 1 (one) hour.

The silicon sheet which had been subjected to graft polymerization was soaked in the solution, and stirred for 1 (one) hour.

Thereafter, the silicon sheet was taken out, soaked in 300 mL of ion exchanged water, and then washed for 30 minutes while being stirred.

0.045 g of PAA-15C (allylamine (free) polymer (Nittobo Medical Co., Ltd.)) was dissolved in 300 mL of ion exchanged water, and a resultant solution was stirred for 10 minutes.

The silicon sheet which had been washed was soaked in the solution, and stirred for 2 hours.

Thereafter, the silicon sheet was taken out, soaked in 500 mL of ion exchanged water, and then washed for 1 (one) day while being gently stirred.

The silicon sheet having the surface to which the amino group was introduced was thus obtained.

### (C. Coating of silicon sheet with silver particles)

"5 mM CDT-Ag" prepared in <1. Preparation of silver particles> was suspended in 100 mL of ion exchanged water, and a resultant solution was subjected to ultrasonic irradiation for 10 minutes.

The silicon sheet (45 mm × 35 mm) having the surface to which the amino group was introduced was soaked in the solution. The solution was then subjected to ultrasonic irradiation for 5 minutes.

Next, the solution was stirred at 40°C for 3 hours. The silicon sheet was then taken out from the solution, and air-dried for 1 (one) day.

In a state of being soaked in ultrapure water, the silicon sheet was subjected to ultrasonic irradiation for 3 minutes so as to be washed. The silicon sheet taken out from the solution was air-dried.

The silicon sheet to which the silver particles were fixed via the amide bond was thus obtained.

### <7-2. Test method>

*Escherichia coli* (*Escherichia coli* Wild W3310), *Pseudomonas aeruginosa* (*Pseudomonas aeruginosa* PA01), *Serratia* (*Serratia marcescens* W124), *Salmonella* (*Salmonella enteritidis* LT2), *Bacillus cereus* (*Bacilliis cereus* W127), *Staphylococcus aureus* (*Staphylococcus aureus* 209P), and *Streptococcus pyogenes* (*Streptococcus pyogenes* W116) were each independently cultured in an LB liquid culture medium.

Next, an aqueous solution which contained such bacteria in concentration of 1×10⁵ cells/mL was prepared from the LB liquid culture medium after culture. Specifically, an aqueous solution was prepared by diluting, with a saline solution (140 mM NaCl), the LB liquid culture medium after the culture. An amount of the saline solution used for dilution was adjusted so that absorbance (OD600) of the aqueous solution was 0.1 at a wavelength of 600 nm. Thus, the aqueous solution having a bacterial concentration of 1×10⁵ cells/mL was prepared.

The aqueous solution (40 µL) containing the bacteria was spread on the surface of the silicon sheet (sterilized with 70% ethanol) to which the silver particles were fixed or on a surface of a silicon sheet (sterilized with 70% ethanol) to which the silver particles were not fixed. Note that the silicon sheet to which the silver particles were not fixed was used to carry out a control test. The silicon sheet on which the aqueous solution was spread was allowed to stand still for 60 minutes or 120 minutes.

Thereafter, the aqueous solution on the silicon sheet was collected. With use of a saline solution (140 mM NaCl), a 10-fold dilute aqueous solution, a 10²-fold dilute aqueous solution, a 10³-fold dilute aqueous solution, a 10⁴-fold dilute aqueous solution, and a 10⁵-fold dilute aqueous solution were prepared from the aqueous solution.

5 µL of each of those dilute aqueous solutions was independently spotted on an LB agar culture medium. The LB agar culture medium was allowed to stand still at 37°C for 24 hours so that the bacteria were cultured.

After culture, colonies which appeared on the LB agar culture medium were counted. Then, a comparison was made between (i) the count of the colonies which were obtained by culturing the each of the dilute aqueous solutions that were prepared from the aqueous solution collected from the silicon sheet to which the silver particles were fixed and (ii) that of the colonies which were obtained by culturing the each of the dilute aqueous solutions that were prepared from the aqueous solution collected from the silicon sheet (Control) to which the silver particles were not fixed. A bactericidal rate of the silicon sheet to which the silver particles were fixed was thus calculated.

### <7-3. Test Results>

Tables 4 and 5 show results of the test.

Note that how to calculate the bactericidal rate shown in Tables 4 and 5 will be described with reference to *Escherichia coli* shown in Table 4.

In a case of *Escherichia coli*, when the silicon sheet (Ag) to which the silver particles were fixed was used, the count of the colonies obtained by culturing the 10³-fold dilute aqueous solution was 26, whereas, when the silicon sheet (Control) to which the silver particles were not fixed was used, the count of the colonies obtained by culturing the 10⁴-fold dilute aqueous solution was 9 (see Table 4).

In accordance with the above test results, the count of the colonies, obtained by culturing the 10³-fold dilute aqueous solution that was prepared from the aqueous solution collected from the silicon sheet (Control) to which the silver particles were not fixed, is calculated as 90 (=9 (count of colonies) ×10 (dilution rate)).

Then, by comparing the counts of the colonies obtained by culturing the 10³-fold dilute aqueous solutions, a survival rate of *Escherichia coli* is calculated as approximately 29% (=26/90×100), and a bactericidal rate for *Escherichia coli* is calculated as approximately 71% (=100-29).

Note that, in the above example, the counts of the colonies obtained by culturing the 10³-fold dilute aqueous solutions were compared with each other. However, it is sufficient to use dilute aqueous solutions which are considered possible to bring out more accurate data.

**[Table 4]**

| Still Standing Time | | 60 minutes | | | | | | |
|---|---|---|---|---|---|---|---|---|
| | | Count of Colonies | | | | | Survival Rate (%) | Bactericidal Rate (%) |
| Dilution Rate | | 10 | 10² | 10³ | 10⁴ | 10⁵ | | |
| *Escherichia coli* | Control | | | | 9 | | 29 | 71 |
| | Ag | | | 26 | | | | |
| *Pseudomonas aeruginosa* | Control | | | | 17 | | 29 | 71 |
| | Ag | | | | 5 | | | |
| *Serratia* | Control | | | | 9 | | 10 | 90 |
| | Ag | | | 9 | | | | |
| *Salmonella* | Control | | | 19 | | | 18 | 82 |
| | Ag | | 35 | | | | | |
| *Bacillus cereus* | Control | | | | 6 | | 1 | 99 |
| | Ag | | 6 | | | | | |
| *Staphylococcus aureus* | Control | | 64 | | | | 6 | 94 |
| | Ag | 38 | | | | | | |
| *Streptococcus pyogenes* | Control | | | | 12 | | 10 | 90 |
| | Ag | | | 12 | | | | |

**[Table 5]**

| Still Standing Time | | 120 minutes | | | | | | |
|---|---|---|---|---|---|---|---|---|
| | | Count of Colonies | | | | | Survival Rate (%) | Bactericidal Rate (%) |
| Dilution Rate | | 10 | 10² | 10³ | 10⁴ | 10⁵ | | |
| *Escherichia coli* | Control | | | | | 9 | 0.1 | 99.9 |
| | Ag | | 9 | | | | | |
| *Pseudomonas aeruginosa* | Control | | | | 17 | | 15 | 85 |
| | Ag | | | 26 | | | | |
| *Serratia* | Control | | | | 9 | | 4 | 96 |
| | Ag | | 37 | | | | | |
| *Salmonella* | Control | | | 14 | | | 2 | 98 |
| | Ag | 26 | | | | | | |
| *Bacillus cereus* | Control | | | | | 6 | 0.01 | 99.99 |
| | Ag | 4 | | | | | | |
| *Staphylococcus aureus* | Control | | 64 | | | | 3 | 97 |
| | Ag | 21 | | | | | | |
| *Streptococcus pyogenes* | Control | | 23 | | | | 7 | 93 |
| | Ag | 17 | | | | | | |

As shown in Tables 4 and 5, it was found that the silicon sheet to which the silver particles of the present example were fixed had antibacterial activity for various bacteria. It was considered that the silicon sheet exhibited the antibacterial activity by such a mechanism that precipitated bacteria came into contact with the silicon sheet and accordingly, an outer surface (for example, a cell membrane) of the bacteria was damaged by the contact.

### <8. Safety test of silver particles-1>

In the present example, safety of the silver particles for a living body was tested in accordance with the growth inhabitation zone method. A method of this test and results of the test will be described below.

First, an LB agar culture medium containing *Escherichia coli* was prepared.

Note that an LB agar culture medium is substantially transparent at an early stage of culture because the count of *Escherichia coli* is low, but is whitish at a late stage of the culture because the count of *Escherichia coli* is high. In a case where there is, on the LB agar culture medium, a zone in which growth of *Escherichia coli* is inhibited, the zone remains substantially transparent even at the late stage of the culture.

To the LB agar culture medium, (i) the silicon sheet (sterilized with 70% ethanol) which was prepared in <7. Antibacterial test of silver particles-3> and to which the silver particles were fixed and (ii) the silicon sheet (sterilized with 70% ethanol) to which the silver particles were not fixed were attached. Note that the silicon sheet to which the silver particles were not fixed was used to carry out a control test. *Escherichia coli* were then cultured at 37°C for 24 hours.

After culture, it was examined whether or not there was, around each of the silicon sheets, a zone in which growth of *Escherichia coli* was inhibited.

In a case of the silicon sheet, serving as a control, to which the silver particles were not fixed, *Escherichia coli* actively grew around the silicon sheet and on part of the LB agar culture medium which part was under the silicon sheet.

On the other hand, in a case of the silicon sheet to which the silver particles were fixed, the growth of *Escherichia coli* was inhibited on part of the LB agar culture medium which part was under the silicon sheet. That is, it was found that, basically, the silicon sheet to which the silver particles of the present example were fixed exhibited the antibacterial activity on its surface.

This indicates that, even in a case where the silicon sheet to which the silver particles of the present example are fixed is transplanted into a living body, an antibacterial effect is not brought about over a wide range of the living body (in other words, a causative substance of the antibacterial effect is not diffused in the living body) and that the antibacterial effect is brought about only in a desired zone (in other words, the silicon sheet to which the silver particles of the present example are fixed is safe for the living body).

### <9. Safety test of silver particles-2>

In the present example, safety of the silver particles for a living body (specifically, an animal cell) was tested. A method of this test and results of the test will be described below.

McCoy cells, each of which is a mouse-derived skin fibroblast, were grown in a petri dish containing a culture medium (DMEM (SIGMA-ALDRICH) containing 10% of FCS).

After the culture medium was discarded by suction with use of a Pasteur pipette, the McCoy cells adhering to a surface of the petri dish was washed with PBS (phosphate buffered saline).

1.5 mL of a trypsin aqueous solution was added to the petri dish. The petri dish was allowed to stand still for 2 minutes in a CO₂ incubator having a CO₂ concentration of 5%. Thus, the McCoy cells adhering to the surface of the petri dish were removed from the petri dish, and the McCoy cells were dissociated from each other.

Thereafter, the McCoy cells contained in the trypsin aqueous solution were observed with use of an inverted microscope so as to confirm that the McCoy cells were dissociated from each other.

The trypsin aqueous solution containing the McCoy cells was transferred into a tube containing 5 mL of a culture medium (DMEM (SIGMA-ALDRICH) containing 10% of FCS).

The tube was subjected to centrifugation at 1,400 rpm for 2 minutes so as to collect the McCoy cells as precipitations.

The McCoy cells were suspended in 1 (one) mL of the culture medium (DMEM (SIGMA-ALDRICH) containing 10% FCS) to prepare a cell suspension.

Next, (i) the silicon sheet (sterilized with 70% ethanol) which was prepared in <7. Antibacterial test of silver particles-3> and to which the silver particles were fixed and (ii) the silicon sheet (sterilized with 70% ethanol) to which the silver particles were not fixed were placed on a bottom of a petri dish. Note that the silicon sheet to which the silver particles were not fixed was used to carry out a control test. The petri dish was filled with 12 mL of the culture medium (DMEM (SIGMA-ALDRICH) containing 10% of FCS).

After the cell suspension (100 µL) was added to the petri dish, the culture medium in the petri dish was stirred so that a cell density was not ununiform.

The petri dish was stored in the CO₂ incubator, having the CO₂ concentration of 5%, for 24 hours for culture. Thereafter, (ii) the McCoy cells which grew in a unit area (1 cm²) of the silicon sheet to which the silver particles were fixed and (ii) the McCoy cells which grew in a unit area (1 cm²) of the silicon sheet to which the silver particles were not fixed were counted under microscope observation. Identical tests were carried out six times, and an average of results of the tests was calculated.

Table 6 shows the results of the tests.

**[Table 6]**

| | Count of Adhering Cells in Unit Area (×10⁵ cells/cm²) | |
|---|---|---|
| | Control | Ag |
| Test 1 | 2.7 | 2.0 |
| Test 2 | 3.3 | 1.8 |
| Test 3 | 2.2 | 4.2 |
| Test 4 | 2.3 | 7.4 |
| Test 5 | - | 2.9 |
| Test 6 | - | 3.1 |
| Average | 2.64 | 3.55 |
| STDEV | 0.46 | 2.05 |

As is clear from Table 6, growth of animal cells was not inhibited on the silicon sheet to which the silver particles of the present example were fixed, as well as the silicon sheet, serving as a control, to which the silver particles were not fixed.

That is, this indicates that the silicon sheet to which the silver particles of the present example are fixed is safe for a living body.

Note that the present invention is not limited to the description of the embodiments, but may be altered within the scope of the claims. An embodiment or example derived from a proper combination of technical means disclosed in different embodiments or examples is also encompassed in the technical scope of the present invention.

### Industrial Applicability

The present invention can be used to make medical devices and the like antibacterial or to eliminate or kill microorganisms on a medical device and the like. More specifically, the present invention can be used as a coating agent of a medical device and the like.

## Claims

1. A method of controlling a particle size of silver particles prepared by aggregating silver ions, the method comprising the steps of:
- in preparing a mixed solution by adding a thiol compound to a solution containing silver ions, adjusting an amount of the thiol compound added to the solution, the thiol compound being represented by a chemical formula HS-R-A (where R is any organic group, and A is any reactive functional group); and
- adding a reducing agent to the mixed solution.

2. The method as set forth in claim 1, wherein a molarity of the thiol compound and a molarity of the silver ions of the mixed solution are adjusted so that the molarity of the thiol compound / the molarity of the silver ions ≥ 1/1000.

3. The method as set forth in claim 1 or 2, wherein the organic group R is an alkane, an alkene, or an alkyne.

4. The method as set forth in any one of claims 1 through 3, wherein the reactive functional group A is a carboxyl group, a sulphonyl group, a phosphate group, an alkoxysilyl group, a carboxylic acid anhydride, an amino group, or a hydroxyl group.

5. Silver particles comprising a chemically-modifying group represented by a chemical formula S-R-A (where R is any organic group, and A is any reactive functional group), the chemically-modifying group binding to at least part of a surface of particles containing silver.

6. The silver particles as set forth in claim 5, wherein the organic group R is an alkane, an alkene, or an alkyne.

7. The silver particles as set forth in claim 5 or 6, wherein the reactive functional group A is a carboxyl group, a sulphonyl group, a phosphate group, an alkoxysilyl group, a carboxylic acid anhydride, an amino group, or a hydroxyl group.

8. An antibacterial agent comprising silver particles recited in any one of claims 5 through 7.

9. The antibacterial agent as set forth in claim 8, wherein a target is a fungus, a Gram-positive bacterium or a Gram-negative bacterium.

10. A complex comprising:
- silver particles recited in any one of claims 5 through 7; and
- a base material to which the silver particles adhere.

11. The complex as set forth in claim 10 wherein the silver particles bind to the base material through a chemical bond via a reactive functional group A.

12. A medical device comprising a complex recited in claim 10 or 11.
